# DEMANDE DE BREVET EUROPEEN

(11) **EP 4 091 455 A1**
(43) Date de publication de la demande: **23.11.2022**
(21) Numéro de dépôt: 22174721.5
(22) Date de dépôt: 20.05.2022
(51) Int. Cl.: A21C 1/00, A21C 1/14, A21D 8/04, C12M 1/34, G01F 23/00, C12M 1/36

(54) **FERMENTEUR ET PROCÉDÉ POUR PRODUIRE DU LEVAIN LIQUIDE**

(30) Priorité: 20.05.2021 BE 202105412
(71) Demandeur: JAC S.A., 4000 Liège (BE)
(72) Inventeur: VAN CAUWENBERGHE, Baudouin, 4000 Liège (BE)
(74) Mandataire: Callewaert, Koen

(57) **Abrégé**

Fermenteur à levain avec une cuve (2) pour préparer du levain liquide, comprenant un capteur pour mesurer la quantité du mélange dans la cuve (2). Le fermenteur (1) comprend un dispositif de contrôle qui permet de stocker une valeur minimale pour la quantité du mélange (16) dans la cuve (2). Le dispositif de contrôle (6) est adapté pour recevoir une valeur de quantité mesurée par le capteur et pour comparer cette valeur de quantité mesurée avec ladite valeur minimale stockée. Une alarme est générée lorsque la valeur de quantité mesurée est égale ou inférieure à la valeur minimale stockée.

## Description

L'invention concerne un procédé pour produire du levain liquide à partir d'un mélange comprenant de l'eau et de la farine qui sont fournis dans une cuve. Ainsi, pour obtenir du levain liquide, on applique des étapes de repos successives séparées l'une de l'autre par une étape d'agitation.

Lors d'une étape d'agitation, le mélange de levain liquide est remué pour homogénéiser ce mélange. Dans une étape de repos on soumet le volume de départ du mélange à une fermentation, de sorte que le volume du levain liquide présent dans la cuve augmente.

Dans les fermenteurs à levain liquide de l'art antérieur, l'étape de repos est arrêté après l'écoulement d'un temps prédéfini et le levain est ensuite remué afin de faire descendre le niveau du levain. Après cette étape d'agitation, le mélange est à nouveau soumis à une étape de repos pour la durée prédéfinie. Cette séquence est répétée selon les prescriptions de la recette de préparation du levain liquide qui est utilisée.

Or, pour une bonne fermentation du levain et pour obtenir un levain avec une bonne qualité aromatique, il est important de minimiser l'agitation du levain. Il s'avère que trop d'agitation affecte négativement le développement des arômes et des ferments. Néanmoins, il est important d'agiter le levain de temps en temps pour homogénéiser le mélange, pour repartir la chaleur dans le liquide et pour maintenir un bon développement des ferments.

Lorsqu'on soutire du levain liquide de la cuve, il est important de maintenir une quantité minimale de levain liquide dans la cuve pour démarrer une nouvelle production de levain. La quantité minimale dépend par exemple de la quantité qu'on veut produire ultérieurement.

Le document FR 2 775 265 décrit un fermenteur dans lequel la quantité minimale de levain à maintenir dans la cuve peut être modifiée en changeant la position angulaire d'une tube d'évacuation courbée présentant une vanne.

Ce système présente, entre autres, l'inconvénient qu'il est laborieux et difficile de nettoyer le tube d'évacuation. De plus, le réglage angulaire nécessite une intervention d'un technicien et la quantité de levain, qu'on peut maintenir dans la cuve, est limitée par la longueur du tube d'évacuation.

L'invention veut proposer un fermenteur qui permet de modifier de manière très facile la quantité minimale qui est maintenue dans la cuve lors de la soutirage du levain sans que ceci entraine des manipulations complexes ou un nettoyage difficile.

A cet effet, le fermenteur présente un capteur de niveau sans contact pour mesurer la quantité de levain présente dans la cuve du fermenteur.

Avantageusement, le capteur de niveau comprend un capteur radar, un capteur ultrason ou un capteur optique.

Suivant une forme de réalisation intéressante, le fermenteur comprend un dispositif de contrôle présentant un moyen d'entrée pour permettre d'introduire manuellement une valeur minimale pour la quantité de levain à maintenir dans la cuve.

L'invention concerne également un procédé de contrôle du prélèvement de levain liquide d'une cuve de préparation de levain liquide dans lequel de la farine et de l'eau sont introduits et un mélange est formé qui est soumis à une fermentation pour former du levain liquide, dans lequel le mélange transformé en levain liquide est prélevé de la cuve au moyens d'une vanne d'évacuation prévue à cet effet.

Dans ce procédé la quantité de levain liquide qui est présent dans la cuve est mesurée lors du prélèvement de levain liquide de la cuve et la quantité mesurée est comparée avec une valeur minimale prédéterminée correspondant à une quantité minimale. Cette quantité minimale de levain liquide dans la cuve est nécessaire pour démarrer une nouvelle production de levain liquide. Lorsque la quantité mesurée du levain liquide dans la cuve est égale ou inférieure à ladite valeur minimale, une alarme est générée et/ou la vanne d'évacuation est fermée automatiquement afin de garder la quantité minimale de levain liquide dans la cuve.

D'autres détails et particularités de l'invention ressortiront de la description donnée ci-après, à titre d'exemple non limitatif, de quelques formes de réalisation particulières de l'invention avec référence aux dessins annexés.
La figure 1 est une coupe verticale schématique d'un fermenteur, suivant l'invention.
La figure 2 est une vue analogue à celle de la figure 1 avec le couvercle du fermenteur ouverte lors de la préparation d'un mélange pour préparer du levain liquide.
La figure 3 est une vue analogue à celle des figures 1 et 2 lorsque la cuve du fermenteur contient un volume de départ d'un mélange pour préparer du levain liquide.
La figure 4 est une vue analogue à celle des figures précédentes à la fin d'une étape de repos.

Dans les différentes figures, les mêmes chiffres de référence se rapportent aux mêmes éléments ou à des éléments analogues.

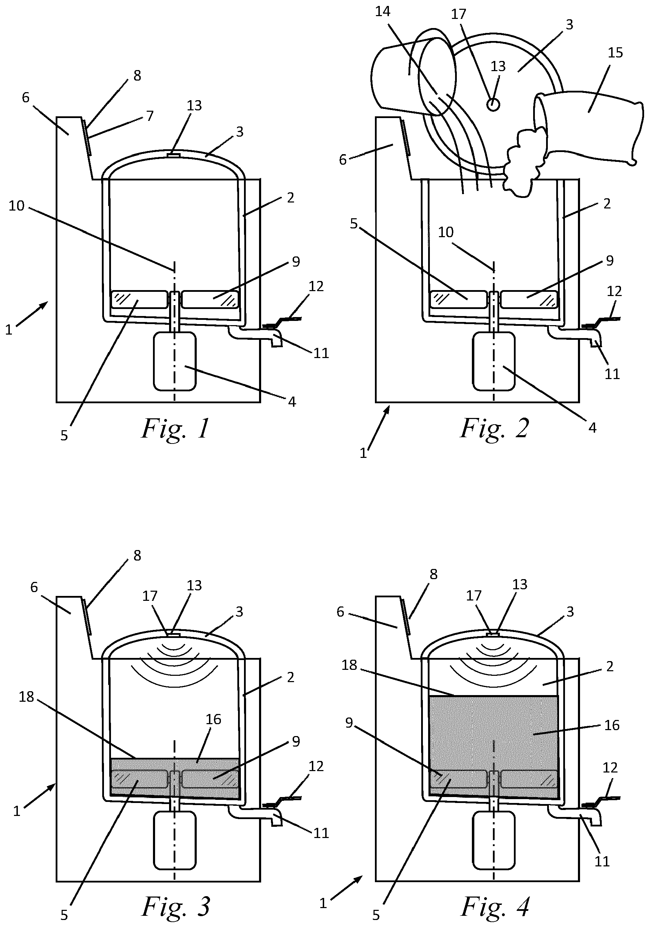

L'invention concerne, en générale, un procédé pour préparer du levain liquide de manière sensiblement automatisée et un fermenteur pour appliquer ce procédé. La figure 1 montre un tel fermenteur 1, suivant une forme de réalisation intéressante de l'invention, comprenant un châssis dans lequel sont prévus une cuve 2 avec un couvercle 3 et un moteur 4 pour entrainer un agitateur 5 présent dans la cuve 2. À sa partie supérieure le fermenteur 1 présente, généralement, un dispositif de contrôle 6 avec, par exemple, un écran 7 et des boutons de commande 8.

De préférence, la cuve 2 présente une section transversale circulaire et l'agitateur 5 comprend une ou plusieurs pales 9 qui peuvent être entrainées autour de l'axe centrale 10 de la cuve 2 pour remuer un liquide présent dans la cuve 2. Ainsi, les pales 9 sont entrainées par le moteur 4 qui est, par exemple, situé en dessous de la cuve 2.

Le fond de la cuve 2 est connecté à un tuyau 11 à travers lequel du liquide peut être prélevé de la cuve 2. Le tuyau 11 peut être fermé ou ouvert par un robinet 12 prévu à cet effet.

Suivant l'invention, le fermenteur 1 comprend un capteur de niveau pour mesurer le niveau d'un mélange présent dans la cuve 2. Dans la forme de réalisation de l'invention représentée dans les figures, ce capteur de niveau 13 est prévu dans le couvercle 3, en particulier, au milieu de celui-ci. Il est évident que ce capteur 13 peut être prévu à d'autres endroits dans le fermenteur 1.

Le levain liquide est préparé à partir d'un mélange comprenant de l'eau et de la farine. Comme le montre la figure 2, de l'eau 14 et de la farine 15 sont versés dans la cuve 2 du fermenteur 1 pour obtenir le mélange à partir duquel le levain est préparé. Eventuellement, des additifs supplémentaires peuvent être ajoutés à ce mélange selon les souhaits de l'opérateur pour modifier les arômes du levain.

Ensuite, l'agitateur 5 est actionné en entrainant les pales 9 autour de l'axe 10 par le moteur 4 pour mélanger et homogénéiser le contenu de la cuve 2. Lorsque le mélange 16 présent dans la cuve 2 est bien mélangé et homogène, l'agitateur 5 est arrêté.

Selon l'invention, une étape de repos est alors appliquée dans laquelle d'abord le volume du mélange 16 présent dans la cuve 2 est déterminé. Ceci est, de préférence, fait au moyen du capteur de niveau 13 sans contact qui est prévu au-dessus de la surface supérieure 18 du mélange 16 présent dans la cuve 2. Dans la forme de réalisation montrée dans les figures, ce capteur de niveau 13 comprend un capteur radar 17. Ainsi, ce capteur permet de déterminer le volume du mélange 16 présent dans la cuve 2 en mesurant la distance entre ce capteur 17 et la surface supérieure 18 du mélange 16. Donc le niveau de la surface supérieure du mélange étant mesuré, ce niveau formant ainsi un niveau de départ correspondant au volume de départ du mélange 16.

Ensuite, une valeur seuil est définie pour le volume du mélange 16, ou, en d'autres termes, pour le niveau de ce volume, correspondant au volume du mélange 16 qu'on souhait obtenir après une étape de repos.

Pour obtenir cette valeur seuil, le volume de départ est, généralement, multiplié par un facteur comprise entre 1,05 et 3. Ce facteur est normalement déterminé par la compétence de l'artisan ou par une recette pour la préparation du levain liquide. De préférence ce facteur est choisi entre 1,15 et 1,85. Dans des applications pratiques de l'invention, un facteur fixe peut éventuellement être programmé dans le dispositif de contrôle 6 du fermenteur 1.

Généralement, ladite cuve 2 présente une forme cylindrique avec une section horizontale circulaire constante. Ainsi, mesurer le niveau du mélange 16 dans la cuve 2 équivaut à la mesure du volume du mélange présent dans la cuve. Le niveau du mélange dans la cuve correspond sensiblement à la distance verticale entre le fond de la cuve 2 et la surface supérieure 18 du mélange 16 présent.

Après la détermination de la valeur seuil pour le volume du mélange 16 fermenté, le niveau de la surface supérieure 18 du mélange 16 est mesuré lors de la fermentation de celui-ci. Lors de cette fermentation, le volume du mélange 16 présent dans la cuve 2 augmente et donc le niveau du mélange 16 augmente. Le mesurage du niveau du mélange 16 peut être effectuée en continu ou en discontinu par le capteur de niveau 13. Lorsque le niveau du mélange 16 est mesuré en discontinue, le niveau est, par exemple, déterminé à des moments successifs.

Au moment où il est établi que le volume du mélange 16, ou donc le niveau de celui-ci, a atteint la valeur seuil suite à la fermentation de ce mélange, l'étape de repos est arrêtée.

Ceci est fait en commençant une étape d'agitation dans laquelle le mélange 16 fermenté est remué. En raison de cette agitation, le volume du mélange 16 diminue.

Cette étape d'agitation est suivie d'une nouvelle étape de repos comprenant déterminer le volume du mélange 16 dans la cuve 2, ce volume formant un nouveau volume de départ, et soumettre le mélange 16 à une fermentation pour que le volume du mélange 16 augmente à nouveau jusqu'à une valeur seuil qui est déterminée en fonction du nouveau volume de départ du mélange. Cette valeur seuil est obtenue par multiplier le nouveau volume de départ avec le facteur susdit.

Ainsi, dans le procédé pour produire du levain liquide à partir du mélange de l'eau 14 et de la farine 15, on applique des étapes de repos successives séparées l'une de l'autre par une étape d'agitation jusqu'à l'obtention d'un levain liquide qui peut être utilisé dans la préparation de produits de viennoiserie et de boulangerie, comme le pain.

Lorsque la production de levain liquide est terminée, la cuve 2 peut éventuellement être refroidie pour bloquer la fermentation du mélange pour stocker le levain.

Le levain liquide peut être soutirée de la cuve 2 à travers le tuyau 11 en ouvrant le robinet 12 jusqu'à ce que la quantité désirée soit obtenue.

Il est important de maintenir une quantité minimale de levain liquide dans la cuve 2. Cette quantité minimale peut servir de mélange de base pour la production de levain dans un procédé de production ultérieur. Ainsi, le niveau du mélange 16 présent dans la cuve 2 est mesuré lorsque du levain liquide est soutirée de la cuve. Ceci est, par exemple, fait par le capteur de niveau 13. Lorsqu'une valeur minimale prédéterminée du niveau du mélange 16 est atteinte dans la cuve 2, le robinet 12 est fermé automatiquement ou une alarme est déclenchée pour indiquer que le soutirage du levain liquide doit être terminé.

La quantité minimale de levain qui est conservé dans la cuve 2 est choisie, par exemple, en fonction du volume de levain qu'on veut produire ultérieurement dans le fermenteur 1. De la farine et de l'eau sont ajoutés au levain restant dans la cuve pour obtenir un nouveau mélange pour la production de levain liquide dans le fermenteur 1 suivant le procédé de l'invention décrit ci-dessus.

Donc le niveau minimale de la quantité de levain liquide à conserver dans la cuve 2 est ajustable par l'opérateur et peut, par exemple, être introduit manuellement dans le dispositif de contrôle 6 afin de déclencher une alarme lorsque ce niveau prédéterminé est atteint par le volume du mélange et détecté par le capteur de niveau 13.

Le fermenteur 1 peut également comprendre un capteur de contact prévu dans la paroi de la cuve 2 à un niveau qui correspond à un volume minimal de levain qui doit être conservé pour assurer le bon fonctionnement du fermenteur 1 lors de production subséquente de levain.

De manière analogue, un capteur de contact, en particulier un capteur de débordement, est éventuellement prévu dans la partie supérieure de la paroi de la cuve 2. Lorsque le niveau du mélange atteint ce capteur, une alarme est déclenchée ou une étape d'agitation est entamée pour empêcher que le liquide déborde la cuve 2. Il va de soi que, alternativement, le capteur de niveau sans contact 13 peut également être utilisé comme capteur de débordement.

La présence du capteur de niveau 13 permet de mesurer la vitesse de montée de la surface du mélange 16 lors de l'étape de repos. Lorsque cette vitesse dépasse une valeur seuil prédéterminée, ladite étape de repos est terminée par le démarrage d'une étape d'agitation.

Le capteur de niveau 13 détermine le volume du mélange 16 en mesurant la distance entre la surface supérieure 18 du mélange 16 et une référence, en particulier un niveau fixe au-dessus du mélange 16. Ce niveau fixe correspond, par exemple, à la position du capteur 13. Ce capteur de niveau 13 peut être formé par n'importe quel capteur qui permet de mesurer le niveau du mélange, comme par exemple un capteur radar 17, un capteur optique, un capteur ultrason, etc. De préférence le capteur 13 est un capteur non-contact qui est prévu à une distance du mélange et au-dessus de celui-ci. Ce capteur permet de mesurer le niveau du mélange par rapport à ledit niveau fixe ou par rapport au niveau de départ du mélange. Ainsi, il est possible de mesurer le niveau du volume du mélange par rapport à ce niveau fixe ou un changement du niveau du mélange par rapport au niveau de départ de celui-ci.

Le fermenteur à levain, suivant l'invention, se caractérise par la présence du dispositif de contrôle 6 qui permet de recevoir une valeur correspondant au niveau de départ du mélange 13.

Ainsi, cette valeur peut être introduit dans le dispositif de contrôle 6 par l'opérateur après que celui-ci a préparé le mélange de départ. A cet effet, le dispositif de contrôle présent un moyen d'entrée, comme par exemple un écran tactile 7 ou des boutons de commande 8, pour entrer ladite valeur de départ dans le dispositif de contrôle 6. Il est également possible que l'opérateur introduit directement la valeur seuil pour le volume du mélange, ou le niveau de celui-ci, qu'on souhaite obtenir à la fin de l'étape de repos.

Mais, de préférence cette valeur du niveau ou du volume de départ du mélange 16, provenant du capteur de niveau 13, est saisie automatiquement par le dispositif de contrôle 6 suite au coopération entre le capteur 13 et le dispositif de contrôle 6. A partir de cette valeur provenant du capteur 13, une valeur seuil est alors générée par le dispositif de contrôle 6 qui correspond au volume fermenté souhaité, ou au niveau de ce volume souhaité, comme décrit plus haut.

Le dispositif de contrôle 6 coopère alors avec le capteur 13 pour mesurer le niveau du mélange lors de l'étape de repos et pour comparer ladite valeur seuil avec la mesure du niveau par le capteur 13. Lorsque qu'il est établi que cette valeur seuil est atteint, le dispositif de contrôle 6 déclenche l'entrainement de l'agitateur 5 afin de terminer l'étape de repos et de démarrer l'étape d'agitation.

Le dispositif de contrôle 6 est également capable de stocker une valeur minimale pour le niveau du mélange 16 dans la cuve 2. Lorsque cette valeur minimale est atteinte par le mélange 16 comme déterminé au moyens du capteur de niveau 13, le dispositif de contrôle génère une alarme ou déclenche la fermeture automatique d'une vanne d'évacuation 12 par laquelle la cuve 2 peut être vidée.

Suivant une forme de réalisation intéressante du fermenteur, suivant l'invention, des moyens sont prévus pour générer un signal d'alerte pour l'opérateur, dès que la quantité mesurée du mélange 16 franchit une quantité d'alerte qui est stockée dans le dispositif de contrôle 6. Donc, lorsque cette quantité mesurée se rapproche de la valeur minimale pour la quantité du mélange 16 et lorsque cette quantité est inférieure à la quantité d'alerte, le signal d'alerte est généré. Cette quantité d'alerte est, par conséquent, supérieure à la quantité minimale à maintenir dans la cuve 2 et inférieure à la quantité de levain préparée avant de la soutirer de la cuve 2.

Il est bien entendu que l'invention n'est pas limitée aux différentes formes de réalisation décrites ci-dessus, mais que d'autres variantes encore peuvent être envisagées sans sortir du cadre de la présente invention, notamment en ce qui concerne la forme de la cuve 2 et la présence et la position de capteurs. Il est, par exemple, possible que autour de la cuve, des moyens de refroidissement ou de réchauffement sont prévus qui coopèrent avec des capteurs thermiques. Le dispositif de contrôle 6 peut, par exemple, comprendre des moyens pour introduire ou pour activer de cycles préprogrammés d'une succession d'étapes d'agitation et de fermentation.

## Revendications

1. Fermenteur à levain avec une cuve (2) pour contenir un mélange comprenant de l'eau (14) et de la farine (15) pour préparer du levain liquide, le fermenteur (1) comprenant un capteur pour mesurer la quantité du mélange dans la cuve (2) et un agitateur (5) entrainé par des moyens d'entrainement qui permet de remuer le mélange présent dans la cuve (2), **caractérisé en ce que** le fermenteur (1) comprend un dispositif de contrôle permettant de stocker une valeur minimale pour la quantité du mélange (16) dans la cuve (2), ce dispositif de contrôle (6) étant adapté pour recevoir une valeur de quantité mesurée par ledit capteur et pour comparer cette valeur de quantité mesurée avec ladite valeur minimale stockée, tandis que le dispositif de contrôle (6) est prévu pour générer une alarme ou pour fermer automatiquement une vanne d'évacuation (12) permettant de vider la cuve (2) lorsque la valeur de quantité mesurée est égale ou inférieure à la valeur minimale stockée.

2. Fermenteur suivant la revendication 1, dans lequel le dispositif de contrôle présent un moyen d'entrée (7,8) pour permettre d'introduire manuellement ladite valeur minimale par un opérateur.

3. Fermenteur suivant la revendication 1 ou 2, dans lequel ledit capteur est un capteur de niveau (13) permettant de mesurer le niveau du mélange dans la cuve (2) pour déterminer la quantité du mélange (16).

4. Fermenteur suivant la revendication 3, dans lequel le capteur de niveau (13) comprend un capteur radar (17), un capteur ultrason ou un capteur optique.

5. Fermenteur suivant la revendication 3 ou 4, dans lequel le capteur de niveau (13) permet de mesurer la distance entre la surface (18) du mélange (16) et un niveau fixe en haut de la cuve (2).

6. Fermenteur suivant l'une quelconque des revendications 1 à 5, comprenant des moyens pour générer un signal d'alerte pour un opérateur, ces moyens coopérant avec le dispositif de contrôle de manière à générer le signal d'alerte lorsque la quantité mesurée franchit une quantité d'alerte lorsque cette quantité mesurée se rapproche de la valeur minimale pour la quantité du mélange (16) dans la cuve (2).

7. Fermenteur suivant l'une quelconque des revendications 1 à 6, dans lequel ledit capteur permet de mesurer ladite quantité du mélange (16) dans la cuve (2) sans être en contact avec le mélange (16).

8. Procédé de contrôle du prélèvement de levain liquide d'une cuve (2) de préparation de levain liquide dans lequel de la farine (15) et de l'eau (14) sont introduits et un mélange est formé qui est soumis à une fermentation pour former du levain liquide, dans lequel le mélange transformé en levain liquide est prélevé de la cuve (2) au moyens d'une vanne d'évacuation (12) prévue à cet effet, **caractérisé en ce que** la quantité de levain liquide présent dans la cuve (2) est mesurée lors du prélèvement de levain liquide de la cuve (2) et la quantité mesurée est comparée avec une valeur minimale prédéterminée correspondant à une quantité minimale nécessaire pour démarrer une nouvelle production de levain liquide dans la cuve (2), lorsque la quantité mesurée est égale ou inférieure à ladite valeur minimale une alarme est générée et/ou la vanne d'évacuation (12) est fermée automatiquement afin de garder ladite quantité minimale de levain liquide dans la cuve (2).

9. Procédé suivant la revendication 8, dans lequel la quantité de levain liquide est mesurée en mesurant le niveau de celui-ci dans la cuve (2).

10. Procédé suivant la revendication 9, dans lequel le niveau du levain liquide est mesurée par un capteur radar (17), un capteur ultrason ou un capteur optique.

11. Procédé suivant l'une quelconque des revendications 8 à 11, dans lequel la quantité de levain liquide présent dans la cuve (2) est mesurée en déterminant la distance entre la surface (18) du mélange (16) et un niveau fixe en haut de la cuve (2).

12. Procédé suivant l'une quelconque des revendications 8 à 12, dans lequel une quantité d'alerte pour le levain liquide contenue dans la cuve (2) est choisie et un signal d'alerte pour un opérateur est généré lorsque la quantité mesurée franchit la quantité d'alerte lorsque cette quantité mesurée se rapproche de la valeur minimale.
